# EUROPÄISCHE PATENTSCHRIFT

(11) **EP 1 309 294 B1**
(45) Veröffentlichungstag und Bekanntmachung des Hinweises auf die Patenterteilung: **27.07.2005**
(21) Anmeldenummer: 01947292.7
(22) Anmeldetag: 18.05.2001
(51) Int. Cl.: A61F 2/28, A61F 2/78

(54) **SUBKUTANES, INTRAMUSKULÄRES LAGER FÜR EIN STARRES TRANSKUTANES IMPLANTAT**
SUBCUTANEOUS, INTRA-MUSCULAR COUPLING FOR A RIGID TRANSCUTANEOUS IMPLANT
LOGEMENT INTRAMUSCULAIRE SOUS-CUTANE POUR IMPLANT TRANSCUTANE FIXE

(30) Priorität: 15.08.2000 DE 10040590
(43) Veröffentlichungstag der Anmeldung: 14.05.2003
(73) Patentinhaber: ESKA Implants GmbH & Co., 23556 Lübeck (DE)
(72) Erfinder: GRUNDEI, Hans, 23556 Lübeck (DE)
(74) Vertreter: Fuchs Mehler Weiss & Fritzsche
(86) Internationale Anmeldenummer: PCT/EP2001/005726
(87) Internationale Veröffentlichungsnummer: WO 2002/013729

(56) Entgegenhaltungen:
- DE-A- 4 338 746
- DE-A- 19 826 638
- DE-C- 19 857 907
- DE-C- 19 931 882
- FR-A- 2 787 018
- SU-A- 1 375 254
- US-A- 3 683 421
- US-A- 3 947 897
- US-A- 4 158 895

## Beschreibung

Die vorliegende Erfindung betrifft ein subkutanes, intramuskuläres Lager für ein starres transkutanes Implantat, welches inkorporal in einem Knochenstumpf zu verankern ist und welches eine exkorporale Kopplungseinrichtung für ein exoprothetisches Standardteil aufweist.

Ein derartiges Implantat zur Versorgung eines oberschenkelamputierten Patienten ist beispielsweise ausführlich in der DE-198 26 638 beschrieben. In den amputierten Femurstumpf wird danach ein Implantat in Form eines Adapters mit einem Stielteil in den intramedullären Raum gesetzt. Dem Stielteil schließt sich ein Zwischenstück an, welches durch die Durchtrittsstelle im Oberschenkelstumpf austritt. Problematisch hierbei ist die Abdichtung des Gliedmaßenstumpfes, da die Durchbruchsstelle aseptisch gehalten werden muß. Des weiteren ist die Adaption des starren Zwischenstückes an die muskuläre Umgebung im Oberschenkelstumpf sowie an die Haut ein kritischer Punkt. Idealerweise muß die Haut und das Muskel- und Bindegewebe gegenüber dem starren Implantat beweglich sein. Diese Anforderung erschwert freilich das Bemühen, die Durchtrittsstelle aseptisch zu halten.

Aus der US 4 158 895 ist ein eingangs erwähntes subkutanes, intramuskuläres Lager bekannt, welches aus einem felxiblen Material besteht und eine Tülle aufweist, die das Implantat distal fest umschließt. Bei diesem Lager ist die Beweglichkeit der das Lager umgebenden Weicliteile gegenüber dem starren Implantat nicht gegeben.

Ein weiterer wichtiger Aspekt ist, daß ein subkutanes Lager eine hohe Schranke gegen eine Verkeimung von außen bilden muß.

Vor diesem Hintergrund ist es die Aufgabe der vorliegenden Erfindung, das gattungsgemäße subkutane Lager so weiterzubilden, daß sich die Weichteile gegenüber dem starren Implantat bewegen können, ohne daß die Durchbruchstelle im Körperstumpfteil einem erhöhten Risiko einer Entzündung ausgesetzt wird.

Gelöst wird diese Aufgabe durch das subkutane, intramuskuläre Lager mit den kennzeichnenden Merkmalen des Anspruches 1. Vorteilhafte Weiterbildungen ergeben sich aus den Unteransprüchen.

Demnach ist vorgesehen, daß das erfindungsgemäße subkutane, intramuskuläre Lager eine intrakorporal anzuordnende Überwurfhülse in Form eines flexiblen Faltenbalges aufweist. Dieser Faltenbalg ist proximal mit einem angeformten Bund in abdichtender Weise mit der Tülle verbunden, und zwar so, daß zwischen der Innenwandung des Faltenbalges und Außenwandung der Tülle ein Hohlraum der Mindestbreite s von 1 mm freibleibt, wobei distal am Faltenbalg ein flexibles Gitternetzwerk angeordnet ist, dem sich distalseitig ein weiteres 2Gitternetzwerk mit einem gegenüber dem flexiblen Gitternetzwerk höheren E-Modul anschließt.

Die Tülle sitzt fest an dem betreffenden Implantatteil und ist beispielsweise homogen mit diesem verklebt, in der Weise, daß dieser Sitz der Tülle auf dem Implantatteil keimfest ist. In diesem Bereich ist die Überwurfhülse in Form des Faltenbalges angeformt, der einstückig mit der Tülle aus beispielsweise Silikon gefertigt sein kann.

Die Ausbildung der Überwurfhülse als Faltenbalg bietet eine sehr gute Beweglichkeit der das Lager umgebenden Weichteile gegenüber dem starren Implantat.

Distal ist das erwähnte flexible Gitternetzwerk an dem Faltenbalg angesetzt, welches ebenfalls bevorzugt aus Silikon bestehen kann und gegebenenfalls einstückig mit dem Faltenbalg ausgebildet ist. Dieses flexible Gitternetzwerk sorgt mit der Zeit nach der Implantation für einen innigen Verbund mit den umgebenden Muskeln.

Dem flexiblen Gitternetzwerk schließt sich distal das erwähnte weitere Gitternetzwerk an, welches besonders bevorzugt aus einer metallischen gewirkten oder gefilzten Wolle aus Titan gefertigt ist. Diese Titanwolle geht dabei eine innige Verbindung mit der Haut des Patienten im Körperstumpf ein, d. h. die Haut sproßt in das Titangeflecht ein.

Das gesamte Lager ist in dem Körperstumpf eingebettet. Lediglich das starre Implantat tritt aus der Tülle des Lagers aus und weist dort die erwähnte Kopplungseinrichtung für ein exoprothetisches Standardteil auf.

Keime oder Schmutzpartikel können nicht zum Knochenstumpf vordringen, sondern werden in dem Hohlraum des Lagers, gebildet durch den Faltenbalg, aufgefangen.

Mit der erfindungsgemäßen Ausbildung des subkutanen, intramuskulären Lagers ist es möglich, einen Patienten mit einem Implantat im Knochenstumpf dauerhaft zu versorgen, ohne daß schwerwiegende Komplikationen wegen einer Sepsis zu befürchten wären. Aufgrund der Verwachsung der Haut mit dem Titangefiecht und aufgrund der Verwachsung des Muskelgewebes mit dem Silikongitternetzwerk wird eine überaus wirksame natürliche Schranke gegen ein Eindringen von Keimen o. ä. in den Körperstumpf aufgebaut.

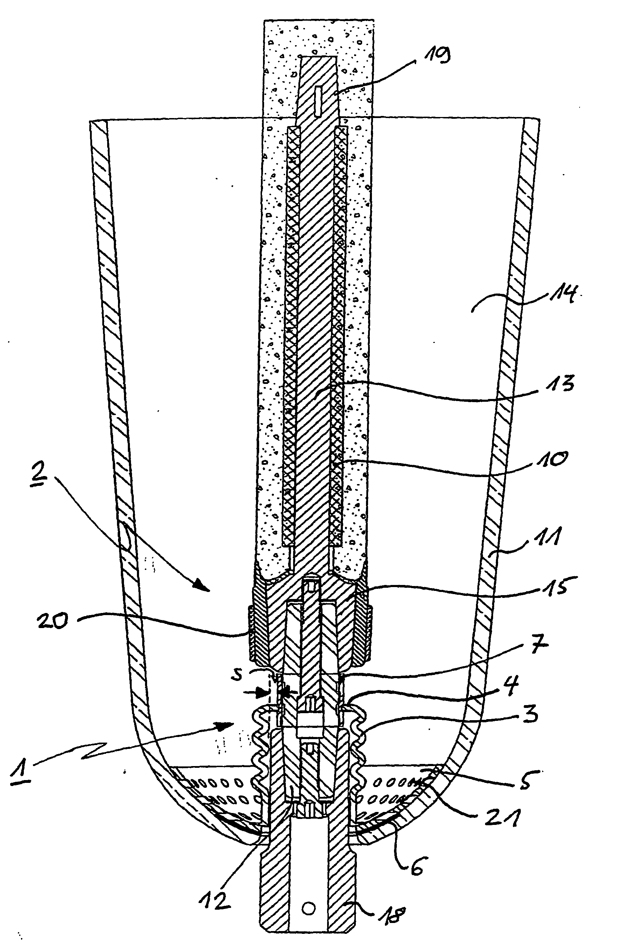

Die Erfindung wird anhand eines Ausführungsbeispiels gemäß der einzigen Zeichnungsfigur näher erläutert.

Darin ist dargestellt ein Oberschenkelstumpf 14 mit dem Femurstumpf 10. In den röhrenförmigen Femurstumpf 10 ist ein Stielteil 13 des Implantates 2 eingeführt. Diesem schließt sich distalseitig ein Überwurfadapter 15 an, welches ein Zwischenstück 12 in Form eines Doppelkonus einfaßt. Dieses Zwischenstück ist das Verbindungsstück zwischen dem Inneren des Oberschenkelstumpfes und der extrakorporalen Umgebung. Es durchtritt dementsprechend die Haut 11 des Oberschenkelstumpfes 14. An seinem außen zu liegen kommenden Abschnitt ist eine exkorporale Kopplungseinrichtung 18 angekoppelt, welche für die Aufnahme eines exoprothetischen Standardteils (nicht dargestellt) dient.

Das vollständig inkorporal, d. h. subkutan und intramuskulär im Oberschenkelstumpf 14 angeordnete Lager 1 besteht im wesentlichen aus einem hier zylindrischen Innenrohr oder Tülle 7, die fest mit dem Implantat 2 verbunden ist und hier mit dem Zwischenstück 12 an dessen zylindrischen Übergangsbereich homogen verklebt ist. Die Klebstelle bildet eine keimfeste Schranke für Keime und Schmutz. Über einen an der Tülle 7 angeformten Bund 4 ist die Überwurfhülse 3 in Form eines Faltenbalges angeordnet. Die Mindestbreite s zwischen der Außenwandung der Tülle 7 und der Innenwandung der Überwurfhülse 3 beträgt mindestens 1 mm und kann beispielsweise bis zu 10 mm betragen.

Distalseitig geht die Überwurfhülse 3 einstückig über in ein flexibles. Gitternetzwerk 5 mit einer großen Anzahl von Durchbrechungen 21, welche nach der Operation im Laufe der Zeit durchsetzt werden von muskulärem Gewebe.

Distalseitig schließt sich dem Gitternetzwerk 5 ein weiteres Gitternetzwerk 6 an, welches einen höheren E-Modul aufweist und besonders bevorzugt aus einer metallischen Wolle aus Titanfasern besteht. Dieses Gitternetzwerk 6 wird nach der Operation im Laufe der Zeit von Hautgewebe durchsprossen.

Der Überwurfadapter 15 ist im übrigen auf seiner Peripherie mit einer offenmaschigen dreidimensionalen Raumnetzstruktur 20 versehen, in welche hinein Knochenpartikel einsprossen und so auf der knöchernen Seite eine weitere Sperre gegen Eindringen von Keimen bildet.

Eine ähnliche Oberfläche trägt im übrigen der Stiel 13 des Implantates 2 im Inneren des Knochenkanals.

Erwähnt sei an dieser Stelle noch das proximale Ende 19 des Stiels 13, welches hier als Steckkonus ausgebildet ist und als Adapter für einen Stiel für ein künstliches Hüftgelenk dienen kann.

## Patentansprüche

1. Subkutanes, intramuskuläres Lager (1) für ein starres transkutanes Implantat (2), welches intrakorporal in einem Knochenstumpf (10) verankerbar ist und welches eine extrakorporale Kopplungseinrichtung (18) für ein exoprothetisches Standardteil aufweist, bestehend aus einem flexiblen Material und aufweisend eine Tülle (7), die das Implantat (2) distal fest umschließt,
**gekennzeichnet durch**,
eine intrakorporal anzuordnende Überwurfhülse (3) in Form eines flexiblen Faltenbalges, der proximal mit einem angeformten Bund (4) in abdichtender Weise mit der Tülle (7) verbunden ist, derart, daß zwischen der Innenwandung des Faltenbalges und Außenwandung der Tülle (7) ein Hohlraum einer Mindestbreite(s) freibleibt, wobei distal am Faltenbalg ein flexibles Gitternetzwerk (5) angeordnet ist, dem sich distalseitig ein weiteres Gitternetzwerk (6) mit einem gegenüber dem flexiblen Gitternetz (5) höheren E-Modul anschließt.

2. Lager (1) nach Anspruch 1, bei dem die Mindestbreite s 1 mm beträgt.

3. Lager (1) nach Anspruch 1 oder 2, welches aus Silikon besteht.

4. Lager (1) nach einem der Ansprüche 1 bis 3, bei dem das flexible Gitternetzwerk (5) aus Silikon besteht.

5. Lager (1) nach einem der Ansprüche 1 bis 4, bei dem das weitere Gitternetzwerk (6) aus einer metallischen gewirkten oder gefilzten Wolle besteht.

6. Lager (1) nach Anspruch 5, bei dem die metallische Wolle aus Titanfasern gebildet ist.

## Claims

1. Subcutaneous, intramuscular bearing (1) for a rigid transcutaneous implant (2), which can be anchored intracorporeally in a bone stump (10) and which has an extracorporeal coupling device (18) for a exoprosthetic standard part, consisting of a flexible material and having a socket (7) which firmly surrounds the implant (2) distally, **characterised by** a bushing (3) to be arranged intracorporeally in the form of flexible bellows which are connected proximally to the socket (7) in sealing manner by a moulded-on collar (4) such that a cavity of a minimum width (s) remains free between the inner wall of the bellows and outer wall of the socket (7), wherein a flexible lattice network (5) is arranged distally on the bellows, to which flexible lattice network (5) a further lattice network (6) having a higher modulus of elasticity with respect to the flexible lattice network (5) is connected on the distal side.

2. Bearing (1) according to claim 1, in which the minimum width s is 1 mm.

3. Bearing (1) according to claim 1 or 2, which consists of silicone.

4. Bearing (1) according to one of claims 1 to 3, in which the flexible lattice network (5) consists of silicone.

5. Bearing (1) according to one of claims 1 to 4, in which the further lattice network (6) consists of a metallic knitted or felted wool.

6. Bearing (1) according to claim 5, in which the metallic wool is formed from titanium fibres.

## Revendications

1. Coussinet sous-cutané et intramusculaire (1) pour un implant transcutané fixe (2), lequel est ancré de manière intracorporelle dans un moignon osseux (10) et lequel présente un dispositif de couplage extracorporel (18) pour une portion exoprothétique standard, composé d'un matériau flexible et présentant un embout (7) qui distalement entoure solidement l'implant (2),
**caractérisé en ce que**
une gaine d'arrêt intracorporelle (3) sous forme d'un raccord à soufflet flexible, qui proximalement est fixée à un collet (4) façonné de manière étanche avec l'embout (7), de telle sorte qu'un espace vide d'une largeur minimale entre la paroi interne du raccord à soufflet et la paroi externe de l'embout (7) soit libéré, dans lequel distalement au raccord à soufflet est placé un grillage flexible (5), qui est raccordé distalement à un grillage supplémentaire (6) présentant un module élevé par rapport au grillage flexible (5).

2. Coussinet (1) selon la revendication 1, dans lequel la largeur minimale atteint 1 mm.

3. Coussinet (1) selon la revendication 1 ou 2, lequel est composé de silicone.

4. Coussinet (1) selon l'une quelconque des revendications 1 à 3, dans lequel le grillage flexible (5) est constitué de silicone.

5. Coussinet (1) selon l'une quelconque des revendications 1 à 4, dans lequel le grillage supplémentaire (6) est composé d'une laine métallique tricotée ou feutrée.

6. Coussinet (1) selon la revendication 5, dans lequel la laine métallique est composée de fibres de titane.
